# EUROPEAN PATENT SPECIFICATION

(11) **EP 3 913 047 B1**
(45) Date of publication and mention of the grant of the patent: **05.07.2023**
(21) Application number: 20305519.9
(22) Date of filing: 19.05.2020
(51) Int. Cl.: C12N 5/0735, C12N 5/0793, C12N 5/077, C12N 5/074

(54) **METHOD FOR GENERATING FUNCTIONAL SKELETAL MUSCLE FIBERS INNERVATED BY MOTONEURONS**
VERFAHREN ZUR HERSTELLUNG VON MIT MOTONEURONEN INNERVIERTEN FUNKTIONALEN SKELETTMUSKELFASERN
PROCÉDÉ POUR GÉNÉRER DES FIBRES DE MUSCLES SQUELETTIQUES INNERVÉES PAR DES MOTONEURONES

(43) Date of publication of application: 24.11.2021
(73) Proprietor: Association Française contre les Myopathies (AFM), 75651 Paris Cedex 13 (FR); Université d'Aix-Marseille, 13007 Marseille (FR); INSERM - Institut National de la Santé et de la Recherche Médicale, 75013 Paris (FR)
(72) Inventor: MAGDINIER, Frédérique, 13013 Marseille (FR); MAZALEYRAT, Kilian, 13010 Marseille (FR)
(74) Representative: Cabinet Becker et Associés

(56) References cited:
- WO-A1-2016/016451
- WO-A1-2019/183597
- JORGE-MIGUEL FAUSTINO MARTINS ET AL: "Self-Organizing 3D Human Trunk Neuromuscular Organoids", CELL STEM CELL, vol. 26, no. 2, 1 February 2020 (2020-02-01), pages 172-186.e6, XP55741346, AMSTERDAM, NL ISSN: 1934-5909, DOI: 10.1016/j.stem.2019.12.007
- KILIAN MAZALEYRAT ET AL: "Multilineage Differentiation for Formation of Innervated Skeletal Muscle Fibers from Healthy and Diseased Human Pluripotent Stem Cells", CELLS, vol. 9, no. 6, 23 June 2020 (2020-06-23), page 1531, XP55739782, DOI: 10.3390/cells9061531

## Description

### FIELD OF THE INVENTION

The present invention relates to a method for the generation of functional skeletal muscle fibers innervated by motoneurons, from pluripotent stem cells.

### BACKGROUND OF THE INVENTION

Protocols aimed at modelling skeletal muscle differentiation have been lagging behind compared to other cell lineages. Since differentiation toward the skeletal muscle lineage remains challenging, modelling human skeletal muscle and neuromuscular disorders has been particularly hindered by a limited number of protocols for generation of mature and functional muscle fibers with sarcolemmal organization.

*In vivo,* skeletal myogenesis involves tightly controlled spatial and temporal cues and most of the current strategies take advantage of the cascade of events of somitogenesis during embryogenesis. Available protocols are either based on induced expression of exogenous myogenic genes such as *PAX3*, *PAX7* and *MYOD1* or use of small molecules that regulates signalling pathways involved in differentiation during embryonic myogenesis, with variable degrees of efficiency. Indeed, regardless of the protocol, one of their commonalities is the duration of the differentiation process and yield. In particular, cultures are difficult to maintain on the long-term and only partially recapitulate the organization and function of mature muscle with a lack of sarcolemmal architecture, expression of adult isoforms of muscle-specific protein and persistence of foetal and embryonic proteins, and low or absence of functionality. Yet, *in vitro* systems able to recapitulate *in vivo* skeletal muscle differentiation are strongly needed for basic research, disease modeling or drug discovery for a wide range of neuromuscular and muscular disorders for which the molecular mechanisms remain unclear or are lacking a therapy

### SUMMARY OF THE INVENTION

The inventors describe herein a novel, reliable and reproducible method for the co-differentiation of pluripotent stem cells (PSCs) into functional skeletal muscle fibers and motor neurons. Advantageously, the presence of both cell types greatly enhances myoblast differentiation, favors maturation but also allows persistence of precursor cells. More importantly, the method allows to fully recapitulate the organization and function of mature skeletal muscle fibers forming neuromuscular junctions (NMJs) with the co-differentiated motor neurons. Compared to a number of other differentiation methods, the method of the invention is simple and does not depend on the transfection of differentiation factors. The method of the invention also allows a long-term culture of differentiated muscle fibers and associated motor neurons, allowed by a continuous renewal of these differentiated cells from a maintained subpopulation of progenitors.

Accordingly, the invention provides a novel method for producing, from PSCs, functional skeletal muscle fibers innervated by motoneurons, comprising the steps of:
a) culturing PSCs in a culture medium comprising a Bone Morphogenic Proteins (BMP) pathway inhibitor and a Wingless and Int-1 (Wnt) pathway inhibitor during 1 to 10 days;
b) switching the cells to a culture medium comprising a BMP pathway inhibitor, Insulin Growth Factor 1 (IGF-1) and Hepatocyte Growth Factor (HGF), and culturing the cells during 1 to 3 days;
c) switching the cells to a culture medium containing IGF1 and lacking HGF and a BMP pathway inhibitor, and culturing the cells during 1 to 8 days;
d) switching the cells to a culture medium containing IGF1 and a γ-secretase and Notch pathway inhibitor, and culturing the cells during 1 to 10 days; and
e) switching the cells to a culture medium suitable to maintain the thus produced functional skeletal muscle fibers innervated by motoneurons.

In a particular embodiment, the Wnt pathway inhibitor of step a) is an indirect GSK3 inhibitor or a direct GSK3 inhibitor, such as a direct GSK3 inhibitor selected in the group consisting of CHIR99021, lithium, 6-bromoindirubin-3-oxime, SB216763, SB415286, CHIR98014, bikinin, TDZD-8, LY2090314 and (2'Z) indirubin. In another particular embodiment, the Wnt pathway inhibitor of step a) is CHIR99021.

In yet another embodiment, the culture medium of step a) further comprises a Rho-associated protein kinase (ROCK) pathway inhibitor, such as thiazovivin Rho kinase inhibitor IV, Fasudil, GSK429286A, or Y-27632.

According to a further embodiment, the BMP pathway inhibitor of steps a) and b) is selected in the group consisting of Bone morphogenetic protein receptor type IB (Bmpr1b, or ALK6) inhibitors, Activin A receptor type I (ACVR1, or ALK2) inhibitors, Activin receptor-Like Kinase 1 (ALK1) inhibitors, Bone morphogenetic protein receptor type II (Bmpr2) inhibitors, Activin receptor type IIA (Acvr2a) inhibitors, and Activin receptor type IIB (Acvr2b) inhibitors; said BMP pathway inhibitor being in particular an inhibitor of both ALK2 and ALK3 receptors. In yet another embodiment, the BMP pathway inhibitor of step a) and b) is LDN193189 (LDN).

According to another embodiment, the γ-secretase and Notch pathway inhibitor of step d) is selected in the group consisting of N-[N-(3,5-Difluorophenacetyl)-L-alanyl]-S-phenylglycine t-butyl ester (DAPT), compound E, tarenflurbil and dibenzazepine. In a particular embodiment, the γ-secretase and Notch pathway inhibitor of step d) is DAPT.

In yet another embodiment, the duration of step a) is of about 6 days, and/or the duration of step b) is of about 1 day, and/or the duration of step c) is of about 4 days, and/or the duration of step d) is of about 5 days.

According to an embodiment, the PSCs of step a) are induced PSCs (iPSCs). In yet another embodiment, iPSCs of step a) are derived from fibroblast, in particular from human fibroblasts. In some embodiments, the iPSCs of step a) are derived from cells of a patient with a neuromuscular disease or a muscular disorder, such as a muscular dystrophy, in particular a muscular dystrophy selected in the group consisting of Duchenne Muscular Dystrophy (DMD), Myotonic Dystrophy (MD), Facio-Scapulo-Humeral Dystrophy (FSHD) and type 2A Limb-Girdle Muscular Dystrophy (LGMD2A).

According to another embodiment, one or more of the culture media of steps a) to e) is(are) serum-free media.

In a further embodiment, the cells are cultured in step a) to e) on a surface coated with laminin, type IV collagen and proteoglycans.

### LEGENDS OF THE FIGURES

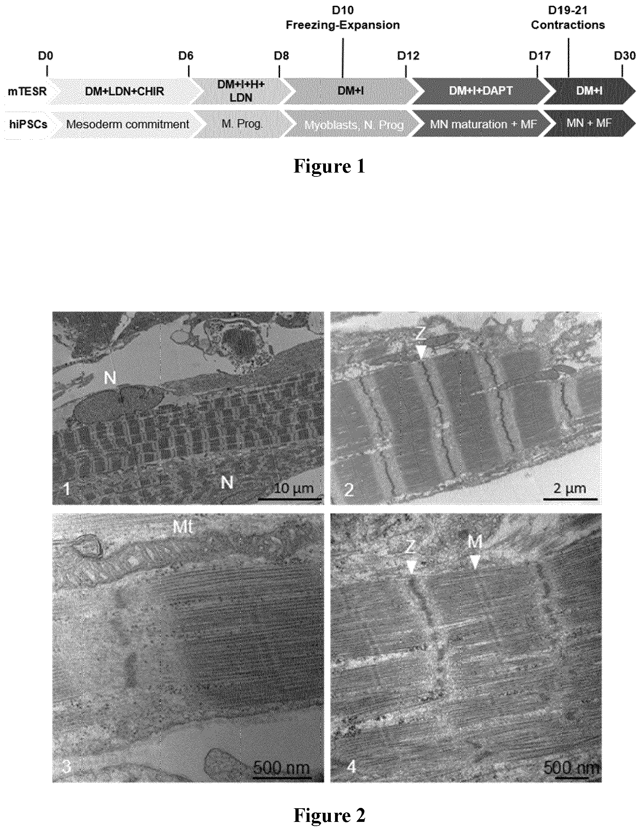

**Figure 1****. Protocol for myogenic differentiation**
   Time line and phase contrast images of cells differentiated from human induced pluripotent stem cells (hiPSCs) at Day 0 (D0), D6, D8, D12, D17 and D30 corresponding to changes in the composition of the medium. Thiazovivin, an inhibitor of the ROCK pathway was added at the time of plating and two days after. The composition of the medium is indicated for the different culture steps. CHIR: CHIR99021; LDN: LDN193189; F: FGF2; I: IGF1; H: HGF; DAPT: N-[N-(3,5-Difluorophenacetyl)-L-alanyl]-S-phenylglycine t-butyl ester. Cells can be frozen between Day 10 and 12 for freezing, storage, further expansion and differentiation. First contractions are visible between day 19 and 21 post differentiation and maintained as long as cells are maintained in culture with daily medium replacement.
**Figure 2****. Ultrastructural features of hiPSCs-derived myofibers by electron microscopy**
   Control cells show complete sarcomeric organization 2 months post differentiation with highly organized myofibrillar patterns and fully mature sarcomeric banding pattern with Z-lines clearly visible (arrows, panels 2; 4), nuclei localized at the periphery of the fibers (panel 1, N) and large mitochondria (panel 3, Mt). Thick filaments are assembled with formation of Z and M lines across Myosin filaments (Panel 4). Z-lines are aligned, forming a clearly visible structural pattern (panels 1;2;4). We also observed 1-bands of Actin filaments and A-bands (panels 2; 4).

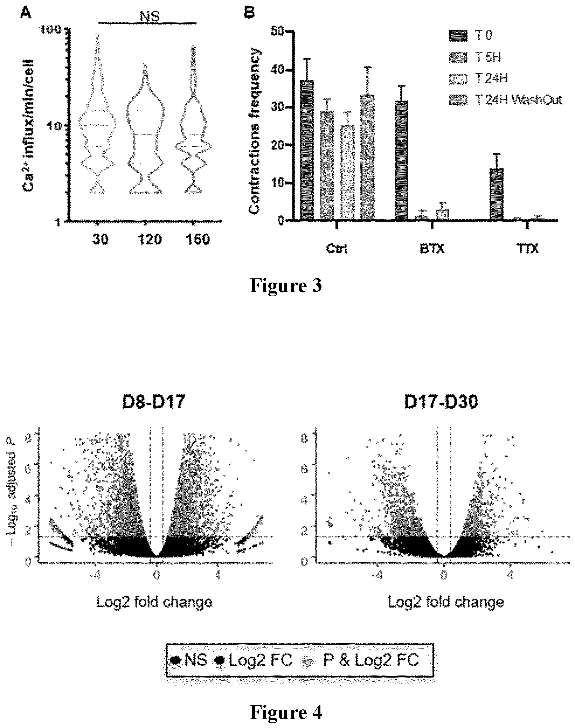

**Figure 3****. Functional validation of hiPSC-derived myofibers**
   **A.** Intracellular Calcium signalling in hiPSC-derived myofibers was measured using an Axio Observed microscope at day 30, day 120 (4 months) and day 150 (5 months) post-differentiation. For each time point, >215 muscle fibers were analyzed. The number of contractions per minute and per fiber is reported as violin plots. No difference was observed in the calcium influx at day 120 and 150 when compared to day 30 (ANOVA multiple comparison test followed by Brown-Forsythe and Welch correction *p*=0.264 and *p*=0.299; respectively). **B.** Contractions were recorded in real time by video microscopy in control cells prior or after addition of Alpha Bungarotoxin (α BTX, responsible for inhibition of acetylcholine receptors) or Tetrodotoxin (TTX, a Sodium channel blocker that irreversibly inhibits action potential) to the cell medium, or after medium replacement and drug removal. Histogram displays the mean number of contractions ± S.D. shown by error bars, prior to drug addition (T0), 5 hrs after (T 5H), 24 hrs after (T 24 H) or 24 hrs after drug removal and medium replacement (T 24H washout). Addition of both drugs irreversibly inhibits cell contraction.
**Figure 4****. Expression shift during hiPSCs differentiation**
   Volcano plots of genes differentially expressed between D8 and D17 (left) and D17-D30 (right panel).
**Figure 5****. Differentially expressed genes (DEGs) between D8-D17 and D17-D30**
   List of selected DEGs between D8-D17 and D17-D30 distributed in four groups according to their expression characteristics.
**Figure 6****. Expression of myogenic markers and persistence of satellite cells over time**
   **A.** Western blot on whole cell extracts performed at different time points post differentiation, D6, 8, 12, 17, 21, 30 but also 90 (3 months), 150 (5 months) and 210 (7 months) days post differentiation using antibodies against PAX7. Human primary myoblasts (M) were used as control. **B.** Western blot on whole cell extracts was performed at different time points post differentiation, D6, 8, 12, 17, 21, 30, 90 (3 months), 150 (5 months) and 210 (7 months) days post differentiation using antibodies against MyH2, MyH3, MyH8 or Desmin. Beta Actin was used as loading control. Representative western blots are presented together with **C.** quantification of ratio between muscle markers and Beta Actin at the different time points. Quantification corresponds to the average of three independent experiments.

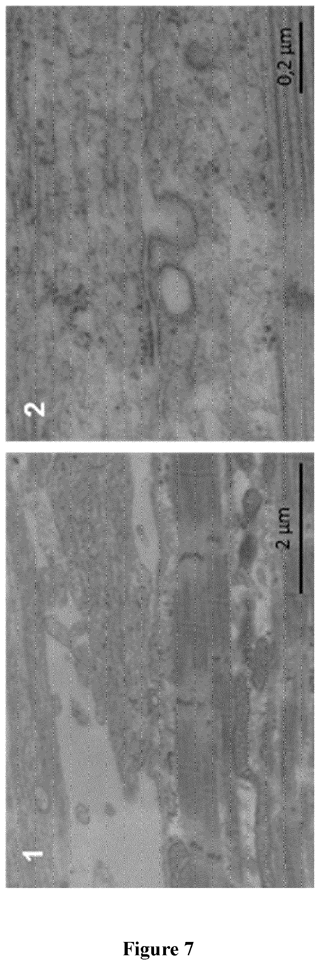

**Figure 7****. Characterization of NMJs**
   Transmission electron microscopy showing the presence of an axon positioned close to a muscle fiber. The basal lamina is visible (photographs 1-2), as well as invagination of the plasma membrane (photograph 2).

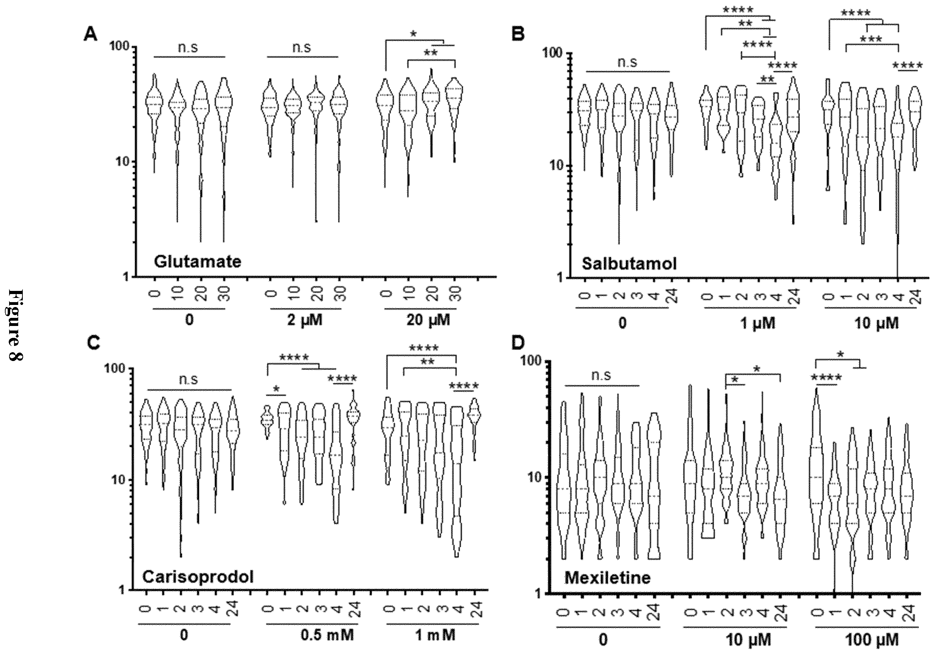

**Figure 8****. Evaluation of the functional response to pharmaceutical drugs**
   **A-D.** We measured the transient Calcium influx in control myofibers prior to drug addition (0 hr) and at different time points after a single addition of different concentrations of each drug. Violin plots display the number of influx per minute and per fiber (n>50). Statistical significance was determined using ANOVA multiple comparison test followed by Brown-Forsythe and Welch correction and comparison to the basal condition (prior to drug addition). **A.** 0; 2 and 20 µM for Glutamate, **B.** 0; 1 and 10 µM for Salbutamol. C. 0; 0.5 and 1 mM for Carisoprodol. **D.** 0; 10 and 100 µM for Mexiletine.

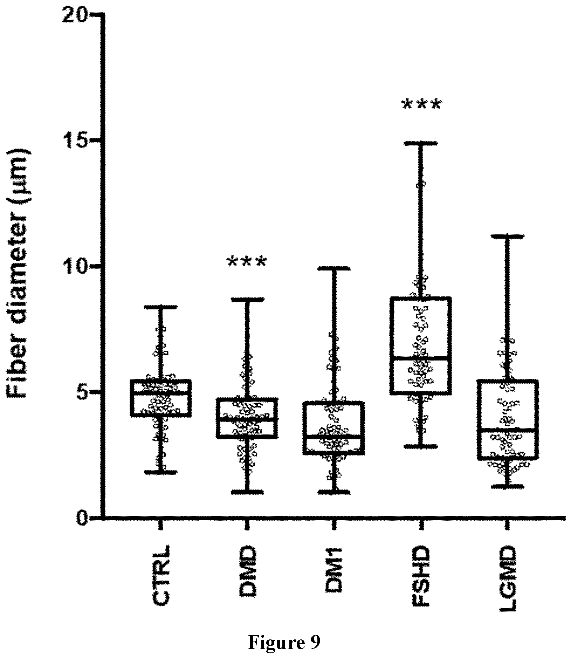

**Figure 9****. Functional phenotyping of hiPSC-derived muscle cells from patients affected with a muscular dystrophy**
   We compared muscle fibers section of Titin-stained fibers in control cells and the four different pathologies (DMD, DM1, FSHD or LGMD2A). For each condition, 500 fibers were analyzed and the section of the fiber was determined at three different positions along each fiber. Box plots displays the mean sizes for the different samples. Values were compared using ANOVA multiple comparison test followed by Brown-Forsythe and Welch correction test; ^{∗∗∗∗}, *p*<0.0001; ^{∗∗∗}, *p*<0.001; ^{∗∗}, *p*<0.01.

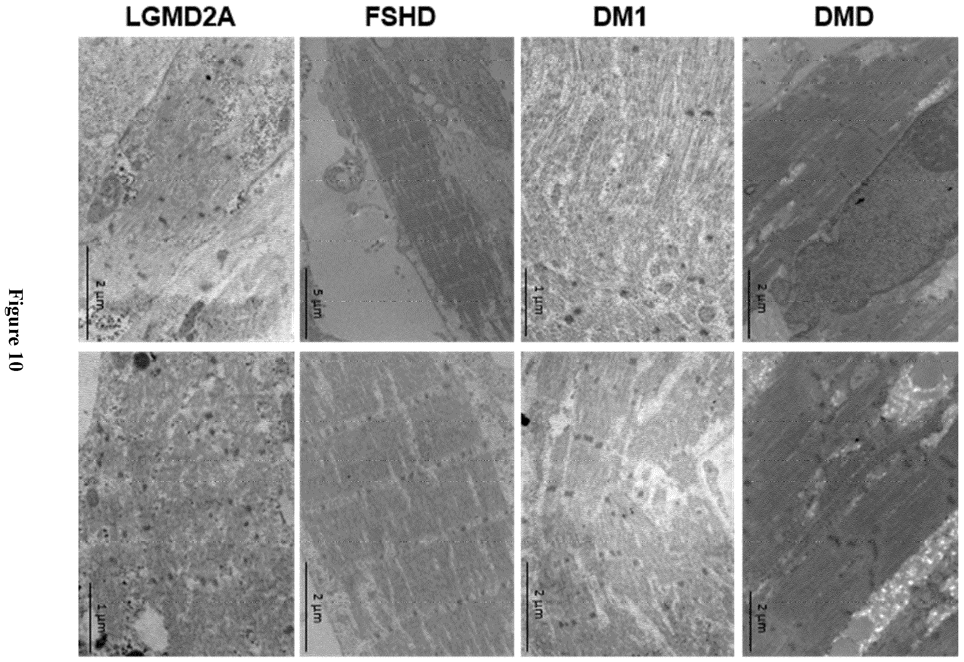

**Figure 10****. Ultrastructural characterization of diseased hiPSC-derived muscle fibers.**
   Ultrastructural features of hiPSC-derived muscle fibers for the different neuromuscular disorder. Electron microscopy was performed 60 days post differentiation. Representative image for DMD, DM1, FSHD and LGMD2A are presented.

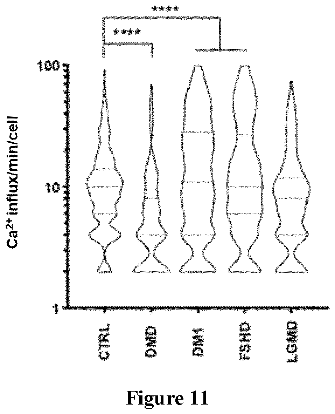

**Figure 11****. Functional characterization of diseased hiPSC-derived muscle fibers**
   Intracellular Calcium signalling in hiPSC-derived myofibers was measured using an Axio Observed microscope at day 45 post-differentiation. For each time point, >180 muscle fibers were analyzed. We report the number of influx recorded per minute and per fiber as violin plots. Data were compared using an ANOVA multiple comparison test followed by Brown-Forsythe and Welch correction. DM1 myofibers present less calcium influx when compared to control (*p*<0,0001) whereas Calcium influx increases in FSHD and DMD myofibers (*p*<0,0001). No difference was observed between control and LGMD2A myofibers (*p*=0,89).

### DETAILED DESCRIPTION OF THE INVENTION

The invention relates to a method for generating functional skeletal muscle fibers innervated by motor neurons. According to the invention, co-differentiation of these cells is carried out by the selective addition of small molecules in a particular sequence.

### Definitions

As used herein, the term "about" used with respect to a number of days of cell culture represents the period of time corresponding exactly to said number of days, for example 24 hours for one day, 48 hours for 2 days, etc. but also a period with a tolerance of +/- 4 hours. For example, about 1 day means 20 to 28 hours, about 2 days means 44 to 52 hours, about 6 days means 140 to 148 hours, about 10 days means 236 to 244 hours, etc.

Furthermore, the term "about" used with respect to a concentration value means +/- 10% of this concentration value, such as +/- 5% of this concentration value.

### Functional muscle fibers

Thanks to the method of the invention, skeletal muscle fibers innervated by motor neurons are generated. As used herein, the expression "functional skeletal muscle fibers" refers to muscle cells that recapitulate key functional aspects of skeletal muscles including their ultra-structural structure, development and contractile apparatus. In particular, the method of the invention may generate skeletal muscle fibers having one or more, preferably all, of the following features:
- they are millimetre-long fibers;
- they exhibit well-organized sarcomeric structure with A-band of myosin filaments, I-bands of actin filaments, M-lines and Z-lines;
- they have a high degree of maturity that can be assessed by the detection of adult isoforms of myosin, such as MYH2 or MYH7;
- they promote the synthesis of extracellular matrix; and/or
- they contain more than one nucleus.

Generated skeletal muscle fibers are "innervated by motor neurons", meaning that they are functionally connected to the motor neurons during the implementation of the method of the invention. The thus generated skeletal muscle fibers and motor neurons are in close proximity and are able to connect with each other to form functional neuromuscular junctions (NMJs), with the development of axons and the presence of synaptic cleft at the surface of muscle fibers. Remarkably, the skeletal muscle fibers generated thanks to the invention can exhibit contractile activity caused by nervous stimuli from the connected motor neurons, and the response to pharmaceutical drugs are consistent with the ones well described for NMJs.

### Pluripotent stem cells

As used herein, the term "pluripotent stem cell" (PSC) denotes a cell that has the ability to self-replicate for indefinite periods and can give rise to every cell types under the right conditions, particularly, the cell types that derive from all three embryonic germ layers-mesoderm, endoderm, and ectoderm.

PSCs may be derived from mammalian cells, in particular human cells. Among PSCs, the most commonly used are embryonic stem cells (ESCs) and induced pluripotent stem cells (iPSCs).

In a particular embodiment of the invention, PSCs used in the invention are ESCs, wherein said ESCs are not produced using a process which involves modifying the germ line genetic identity of human beings, which involves use of a human embryo for industrial or commercial purposes, or which involves destruction of a human embryo.

In a particular embodiment, the PSCs used in the practice of the invention are iPSCs obtained as previously described in the state of the art. The practitioner can in particular refer to Takahashi and Yamanaka, 2006 and 2007. iPSCs useful in the practice of the invention may be derived from a mammalian, in particular from a human subject. The cells used for obtaining iPSCs, here referred as source cells, may be obtained at any stage of development, and are in particular of fetal origin, or derived from cells obtained from a young (for example a children) or adult subject. iPSCs may be derived from any source tissue, as is well known to those skilled in the art. For example, iPSCs may be obtained from blood, skin, muscle, umbilical cord, etc. In a particular embodiment, iPSCs are generated from primary fibroblasts, such as from primary skin fibroblasts, in particular human primary skin fibroblasts. In another particular embodiment, human iPSCs are obtained after infection of these cells with a lentivirus encoding the four Yamanaka's factors OCT4, KLF4, SOX2 and c-MYC (OKSM). Commercial kits useful for generating iPSCs are available such as the STEMCCA-OKSM polycistronic vector from Millipore. Of course, other methods for generating iPSCs are known to a person skilled in the art, for example by using Sendai virus or other non-integrative vectors, that notably reduce the risk of mutations and prolonged expression of the exogenous genes.

In the practice of the present invention iPSCs may be generated from cells of healthy subjects or from subjects having a disease. In a particular embodiment, iPSCs are derived from cells of subjects with neuromuscular or muscular pathologies. For example, iPSCs can be derived from cells of patients with a muscular dystrophy (MD) or neuromuscular disease (NMD). In a particular embodiment, the iPSCs are derived from cells of patients with amyotrophic lateral sclerosis, Charcot-Marie-Tooth disease, Forbes disease, mitochondrial myopathy, congenital myasthenic syndrome, Dejerine-Sottas disease, Friedreich's ataxia, Lactate dehydrogenase deficiency, multiple sclerosis, glycogen storage disease type II, spinal muscular atrophy or rasopathies, metabolic myopathies or myopathies for which causative genes are unknown. In a further particular embodiment, iPSCs used in the method are derived from patients with a muscular dystrophy, in particular a muscular dystrophy selected in the group consisting of Duchenne muscular dystrophy, Becker muscular dystrophy, congenital muscular dystrophy, Emery-Dreifuss muscular dystrophy, distal muscular dystrophy, myotonic dystrophy, facio-scapulo-humeral dystrophy, type 2A limb-girdle muscular dystrophy and oculopharyngeal muscular dystrophy.

Before implementation of the method of the present invention, PSCs may be cultured in a medium different from the medium which is used in co-differentiation steps. For example, iPSCs can be cultured and maintained in mTeSR^{™} (STEMCELL technologies), StemMACS^{™} (Myltenyi Biotec) or StemPro^{®} (Thermo Fisher) medium, on a suitable support, for example on dishes coated with Matrigel or any other suitable coating. In a particular embodiment, the cells used in the method of the invention are cultured on a coated surface, in particular on a surface coated with laminin, collagen type IV and proteoglycans, such as Matrigel^{™} (BD Biosciences), a surface coated with vitronectin, or a Geltrex^{™} matrix (Thermo Fisher).

Furthermore in order to initiate the cell culture according to the method described in the following, PSCs may be mechanically dissociated, thus allowing clumps of cells to be plated for initiating the cell culture procedure. Mechanical dissociation avoids the use of chemical agents that could interfere with the PSCs and potentially inhibit the initial step of the co-differentiation procedure.

### Method for generating skeletal muscle fibers innervated with motor neurons

The invention provides a method for producing, from PSCs, functional skeletal muscle fibers innervated by motoneurons, comprising the steps of:
a) culturing PSCs in a culture medium comprising a BMP pathway inhibitor and a Wnt pathway inhibitor during 1 to 10 days;
b) switching the cells to a culture medium comprising a BMP pathway inhibitor, IGF 1 and HGF, and culturing the cells during 1 to 3 days;
c) switching the cells to a culture medium containing IGF1 and lacking HGF and a BMP pathway inhibitor, and culturing the cells during 1 to 8 days;
d) switching the cells to a culture medium containing IGF1 and a γ-secretase and Notch pathway inhibitor, and culturing the cells during 1 to 10 days; and
e) switching the cells to a culture medium suitable to maintain the thus produced functional skeletal muscle fibers innervated by motoneurons.

Unless otherwise specified, the practice of the present invention implements general techniques in cell culture which are outlined, *inter alia,* in Large Scale Mammalian Cell Culture (Hu et al., 1997); Serum-free Media (K. Kitano, 1991); and Large Scale Mammalian Cell Culture (Spier et al., 1991).

For further elaboration of general techniques useful in the practice of this invention, the practitioner can refer to standard textbooks and reviews in cell biology, tissue culture, and embryology. Included are *inter alia* "Teratocarcinomas and embryonic stem cells: A practical approach" (E. J. Robertson, ed., IRL Press Ltd. 1987); "Guide to Techniques in Mouse Development" (P. M. Wasserman et al. eds., Academic Press 1993); "Embryonic Stem Cells: Methods and Protocols" (Kursad Turksen, ed., Humana Press, Totowa N.J., 2001); "Embryonic Stem Cell Differentiation in Vitro" (M. V. Wiles, Meth. Enzymol. 225: 900, 1993); "Properties and uses of Embryonic Stem Cells: Prospects for Application to Human Biology and Gene Therapy" (P. D. Rathjen et al., al., 1993). Differentiation of stem cells is reviewed, e.g., in Robertson. 1997. Meth Cell Biol 75: 173; Roach and McNeish. 2002. and Methods Mol Biol 185: 1-16.

Any medium suitable to cultivate muscle cells and neurons may be used as a basal culture medium in the practice of the present invention for PSC differentiation. For example, the basal culture medium may be DMEM (Dubelcco's modified essential medium), NPBM (neuronal progenitor cell basal medium) or liquid neurobasal-A medium. In addition to the specific factors described herein used for generating skeletal muscle fibers innervated by motor neurons, which are further described in the following sections, other constituents may be included in the basal medium. Illustrative constituents include N2 supplement, B27 supplement, NEAA supplement, Glutamax supplement, insulin, transferrin, selenium and glutamine. The basal medium may be further supplemented with serum (for example FCS) and/or antibiotics, although a serum-free and/or antibiotic-free culture medium may be more suitable in certain embodiments. In a particular embodiment, the basal culture medium used in the method of the invention is a serum-free medium.

The following sections describe in details each of steps a) to e) of the method of the present invention.

### Step a)

The first step of the method corresponds to the initiation of the differentiation of the cells of interest. It is carried out by the dual modulation of both Wingless and Int-1 (Wnt) and Bone Morphogenic Proteins (BMP) pathways. During this step, PSCs are cultured in a medium comprising a BMP pathway inhibitor and a Wnt pathway inhibitor. In a particular embodiment, the culture medium used for this step is renewed frequently, such as daily or once every two days.

As used herein, the expression "BMP pathway inhibitor" refers to a molecule introduced in the extracellular medium that can inhibit the BMP pathway. This inhibition may more particularly be triggered by the interaction of the molecule with a receptor or a ligand that mediates the BMP pathway signalling through the phosphorylation of the Smad1, Smad5 and Smad8 proteins ("Smad" being the abbreviation referring to the homologies to the *Caenorhabditis elegans* SMA ("small" worm phenotype) and Drosophila MAD ("Mothers Against Decapentaplegic") family of genes). Furthermore, several receptors and ligands that constitute extracellular targets inhibiting BMP pathway are known to the person skilled in the art (Antebi et al. 2017; Borok et al. 2020). In a particular embodiment, the BMP pathway inhibitor is a molecule that inhibits at least one of the following receptors involved in the BMP pathway: Bone morphogenetic protein receptor type IA (Bmprla, or ALK3), Bone morphogenetic protein receptor type IB (Bmpr1b, or ALK6), Activin A receptor type I (ACVR1, or ALK2), Activin receptor-Like Kinase 1 (ALK1), Bone morphogenetic protein receptor type II (Bmpr2), Activin receptor type IIA (Acvr2a), or Activin receptor type IIB (Acvr2b). In a further particular embodiment, the BMP pathway inhibitor is capable of inhibiting at least two receptors involved in the BMP pathway, such as at least two of the above-detailed list. In a particular embodiment, the BMP pathway inhibitor is selected in the list consisting of 4-[6-[4-(1-Methylethoxy)phenyl]pyrazolo[1,5-a]pyrimidin-3-yl]-quinoline (DMH1), 4-[6-[4-[2-(4-Morpholinyl)ethoxy]phenyl]pyrazolo[1,5-a]pyrimidin-3-yl]-quinoline (DMH2), 3-[6-Amino-5-(3,4,5-trimethoxyphenyl)-3 -pyridinyl] -phenol (K02288), 5 -[6-(4-Methoxyphenyl)pyrazolo [1,5 -a]pyrimidin-3-yl]-quinoline (ML347), 5-[6-[4-(1-Piperazinyl)phenyl]pyrazolo[1,5-a]pyrimidin-3-yl]-quinoline (LDN 212854), 1-(4-(6-Methyl-5-(3,4,5-trimethoxyphenyl)pyridin-3-yl)phenyl)piperazine (LDN 214117), 4-[6-[4-(1-piperazinyl)phenyl]pyrazolo[1,5-a]pyrimidin-3-yl]-quinoline or a salt thereof, such its hydrochloride salt, also known as LDN193189 (LDN). In a particular embodiment, the BMP pathway inhibitor may inhibit both ALK2 and ALK3 receptors. In a further particular embodiment, the BMP inhibitor is LDN. In yet another embodiment, LDN is added in the culture medium of step a) at a final concentration of 0.1 - 3 µM, in particular at a final concentration of 0.3 - 0.7 µM and more particularly at a final concentration of about 0.5- µM.

As used herein, the term "Wnt pathway inhibitor" refers to a molecule introduced in the extracellular medium and capable of directly or indirectly inhibiting the Wnt pathway. In a particular embodiment, the Wnt pathway inhibitor is a direct or indirect GSK3 inhibitor. Illustrative indirect GSK3 inhibitors include agonists of Frizzled (FZ) receptor capable of triggering the canonical Wnt pathway. In a particular embodiment, the indirect GSK3 inhibitor is a Wnt pathway inhibitor such as 2-[(6-Chloro-7-cyclopropylthieno[3,2-d]pyrimidin-4-yl)thio]acetic acid (LP922056), N-(6-(3-(Cyclopropanesulfonamido)phenyl)-1H-indazol-3-yl)isobutyramide (SCG-AAK-1.1), or 5-(Phenylsulfonyl)-N-4-piperidinyl-2-(trifluoromethyl)benzene sulfonamide hydrochloride (WAY 316606). In an alternative embodiment, the indirect GSK3 inhibitor is a protein of the Wnt family, preferably a recombinant Wnt protein such as Wnt 3a, Wnt1 or Wnt 7c. In an alternative embodiment, the Wnt pathway inhibitor is a direct GSK3 inhibitor. In a particular embodiment, the direct GSK3 inhibitor is selected from the group consisting of: lithium, 6-bromoindirubin-3-oxime (BIO), 3-(2,4-Dichlorophenyl)-4-(1-methyl-1H-indol-3-yl)-1H-pyrrole-2,5-dione (SB216763), 3-[(3-Chloro-4-hydroxyphenyl)amino]-4-(2-nitrophenyl)-1H-pyrrol-2,5-dione (SB415286), *N*-6-[2-[[4-(2,4-Dichlorophenyl)-5-(1*H*-imidazol-1-yl)-2-pyrimidinyl]amino]ethyl]-3-nitro-2,6-pyridinediamine (CHIR98014) 6-[[2-[[4-(2,4-Dichlorophenyl)-5-(5-methyl-1H-imidazol-2-yl)-2-pyrimidinyl]amino]ethyl]amino]-3-pyridinecarbonitrile (CHIR99021), 4-((5-Bromo-2-pyridinyl)amino)-4-oxobutanoic acid, 4-[(5-Bromo-2-pyridinyl)amino]-4-oxo-butanoic acid (bikinin), 4-Benzyl-2-methyl-1,2,4-thiadiazolidine-3,5-dione (TDZD-8), 3-[9-Fluoro-2-(piperidin-1-ylcarbonyl)-1,2,3,4-tetrahydro[1,4]diazepino[6,7,1-hi]indol-7-yl]-4-imidazo[1,2-a]pyridin-3-yl-1*H*-pyrrole-2,5-dione (LY2090314), 5-ethyl-7,8-dimethoxypyrrolo[3,4-c]isoquinoline-1,3-dione (3F8), 1-(7-Methoxyquinolin-4-yl)-3-[6-(trifluoromethyl)pyridin-2-yl]urea (A-1070722), N-[(4-Methoxyphenyl)methyl]-N'-(5-nitro-2-thiazolyl)urea (AR-A 014418), TC GSA, TC S2002, 3-(1,3-Dihydro-3-oxo-2H-indol-2-ylidene)-1,3-dihydro-2H-indol-2-one; [Δ2,3'-Biindoline]-2',3-dione ((2'Z) indirubin). In a further particular embodiment, the Wnt pathway inhibitor used in the invention is an ATP-competitive GSK3 inhibitor such as BIO, SB216763, SB415286, bikinin, (2'Z) indirubin, LY2090314, 3F8, A-1070722, AR-A 014418, TC GSA, TC S2002, CHIR98014 or CHIR99021. In yet another embodiment, the GSK3 inhibitor is CHIR99021. In a further embodiment, the Wnt pathway inhibitor is CHIR99201, added in the culture medium of step a) at a final concentration of 0.5 - 10 µM, in particular at a final concentration of 1 - 5 µM, such as at a final concentration of about 3 µM.

Step a) is implemented for a duration comprised between 1 to 10 days, in particular for a duration between 4 to 8 days, and more particularly for a duration of about 6 days.

In a particular embodiment, the culture medium used in step a) further comprises a Rho-associated protein kinase (ROCK) pathway inhibitor, in particular a ROCK pathway inhibitor selected from the group of thiazovivin, Rho kinase inhibitor IV, Fasudil, GSK429286A and Y-27632. The addition of a ROCK pathway inhibitor advantageously improves the differentiation yield. In a further particular embodiment, the ROCK pathway inhibitor is thiazovivin. In a further particular embodiment, the ROCK pathway inhibitor is added in the culture medium of step a) at a final concentration of 1 - 100 µM, in particular at a final concentration of 5 - 50 µM, such as a final concentration of 5 - 20 µM, and more particularly at a final concentration of about 10 µM.

In a particular embodiment, the culture medium used in step a) may be supplemented with insulin, transferrin, sodium selenite and/or sodium pyruvate, in particular in the relative proportions (1 X) of the commercially available Insulin-Transferrin-Selenium-Sodium Pyruvate (ITS-A) supplement, such as thrITS-A marketed by Life Technologies. Such an Insulin-Transferrin-Selenium supplementation permits substantial reduction in serum requirement.

### Step b)

In step b), the culture medium is replaced by a new culture medium comprising a BMP pathway inhibitor suitable for step a), in particular the BMP pathway inhibitor used in step a), Insulin-like Growth Factor 1 (IGF-1) and Hepatic growth factor (HGF). Thus, in step b) the culture medium is devoid of the Wnt inhibitor implemented in step a). In a particular embodiment, the culture medium of step b) further comprises β mercapto-ethanol.

In a particular embodiment, the BMP pathway inhibitor is LDN or DMH2, in particular LDN. In yet another particular embodiment, LDN is used in the culture medium of step b) at a final concentration of 0.1 - 3 µM, in particular at a final concentration of 0.3 - 0.7 µM and more particularly at a final concentration of about 0.5 µM.

In a particular embodiment, IGF-1 is added in the culture medium of step b) at a final concentration of 1 - 10 ng/mL, in particular at a final concentration of 2 - 6 ng/mL, and more particularly at a final concentration of about 4 ng/mL.

In a particular embodiment, HGF is added in the culture medium of step b) at a final concentration of 1 - 50 ng/mL, in particular at a final concentration of 5 -15 ng/mL, and more particularly at a final concentration of about 10 ng/mL.

Step b) is implemented for a duration comprised between 1 to 3 days, in particular for a duration of about 1 day.

### Step c)

In step c), the culture medium is replaced by a new culture medium comprising IGF-1. Thus, in step c) the culture medium is devoid of both a BMP pathway inhibitor and HGF. In a particular embodiment, IGF-1 is added in the culture medium of step c) at a final concentration of 1 - 10 ng/mL, in particular at a final concentration of 2 - 6 ng/mL, and more particularly at a final concentration of about 4 ng/mL.

Step c) is implemented for a duration comprised between 1 to 8 days, in particular for a duration of 2 to 6 days, and more particularly for a duration of about 4 days. Advantageously, after step c), the cells can be dissociated to be frozen according to classical techniques of the art. In a particular embodiment, cells of step c) are enzymatically or mechanically dissociated, in particular enzymatically dissociated, such as by accutase treatment, in order to be frozen. According to this particular embodiment, cells of step c) that have been frozen can be thawed later to be amplified and/or used in the following step of the method of the invention.

### Step d)

In step d), the culture medium is replaced by a new culture medium comprising IGF-1 and a γ secretase and Notch pathway inhibitor. In a particular embodiment, the culture medium of step d) further comprises β mercapto-ethanol. In a particular embodiment, the culture medium can be renewed frequently during step d), such as every day, or every two days, in particular every day, using a culture medium with the same composition.

In a particular embodiment, IGF-1 is added in the culture medium of step d) at a final concentration of 1-10 ng/mL, in particular at a final concentration of 2 - 6 ng/mL, and more particularly at a final concentration of about 4 ng/mL.

As used herein, the expression "γ secretase and Notch pathway inhibitor" refers to a molecule introduced in the extracellular medium capable of inhibiting the γ secretase cleavage of Notch protein or blocking the activity of the released intracellular domain of the Notch protein (NICD), thereby inhibiting the NICD-induced regulation of gene expression within the cell. In a particular embodiment, the γ secretase and Notch pathway inhibitor is a γ secretase inhibitor, in particular a non-toxic γ secretase inhibitor. In a particular embodiment the γ secretase inhibitor is selected from the group consisting of: N-[N-(3,5-Difluorophenacetyl)-L-alanyl]-S-phenylglycine t-butyl ester (DAPT), N-[(1S)-2-[[(3S)-2,3-dihydro-1-methyl-2-oxo-5-phenyl-1*H*-1,4-benzodiazepin-3-yl]amino]-1-methyl-2-oxoethyl]-3,5-difluoro-benzeneacetamide (compound E), (2R)-2-(3-Fluoro-4-phenylphenyl)propanoic acid (tarenflurbil) and N-[(1S)-2-[[(7S)-6,7-Dihydro-5-methyl-6-oxo-5H-dibenz[b,d]azepin-7-yl]amino]-1-methyl-2-oxoethyl]-3,5-difluoro-benzeneacetamide (dibenzazepine). In a particular embodiment, the γ secretase inhibitor used in step d) is DAPT. In a more particular embodiment, DAPT is added in the culture medium of step d) at a final concentration of 1 - 100 µM, in particular at a final concentration of 5 - 50 µM, and more particularly at a final concentration of about 10 µM.

Step d) is implemented for a duration comprised between 1 to 10 days, in particular for a duration of 3 - 7 days, and more particularly for a duration of about 5 days.

### Step e)

In step e), the culture medium is replaced by a new culture medium comprising IGF-1. Thus, in step e) the culture medium is devoid of both a γ secretase and Notch pathway inhibitor. During this step, the culture medium can be renewed frequently, such as every day or every two days using a culture medium.

In a particular embodiment, in situations where muscle differentiation is delayed or where a poor enrichment in muscle fiber is observed, HGF and Basic Fibroblast Growth Factor (FGF2) can be added to the medium containing IGF1 of step e) for 1 to 10 days, such as for 3 to 8, in particular for about 7 days. Then, the cells are cultured in a new culture medium comprising IGF-1, but devoid of HGF and FGF2.

Step e) is implemented for a duration that can be extended indefinitely. In a particular embodiment, the duration of step e) is at least 1, 2, 3, 4, 5, 6, 7, 8, 9, 10, 11 or 12 months, or even more than 12 months.

### Skeletal muscle fibers innervated by motor neurons

In another aspect, not being part of the invention, the disclosure relates to skeletal fibers innervated by motor neurons, obtainable by the method described herein.

### Uses of the muscle fibers innervated by motor neurons

In a particular embodiment, the muscle fiber innervated by motor neurons according to the disclosure but not being part of the invention are used as a model to explore muscle development or behavior in a normal or pathological context.

In another aspect not being part of the invention, the disclosure relates to a method of screening the effect of a compound on muscle fibers and motor neurons. In particular, the disclosure relates to a method of screening for a compound useful in the function of the muscle, comprising the treatment of the muscle fibers innervated by motor neurons according to the invention with a test compound, and the determination of the effect of the test compound on function.

In another aspect, the disclosure not being part of the invention relates to a method for investigating muscle wasting associated with a disease. In a particular embodiment, the disease is selected from muscular dystrophies and neuromuscular diseases, as those disclosed above. In yet another embodiment, the disease is cachexia, muscle ageing or sarcopenia.

The invention will now be illustrated with reference to the following non-limiting examples.

### EXAMPLES

### Material and methods

### Cell lines

The iPSCs representing physiological conditions was derived from primary fibroblasts of a healthy subject (Ref AG08498) obtained from the Coriell Institute and previously described (Ortiz-Vitali et al. 2019). 14586 hiPSCs were derived from a patient with a severe clinical FSHD carrying two normal 4q35 alleles but a heterozygote mutation in the *SMCHD1* gene (c.573A>C ; p.Q193P; FSHD2, male, age 67 at sampling) causing a lack of activity of the ATPase domain (2). hiPSCs for DMD (GM25313, male, age 13 at sampling, Ex45 del) and DM1 (GM24559, female, age 2 at sampling, CTG repeat of 1600 in the *DMPK* gene) were purchased from the Coriell Institute. Primary fibroblasts from a patient affected with limb girdle muscular dystrophy (LGMD2A, GM03932, Male, age 67 at sampling) were obtained form the Coriell Institute and reprogrammed as described above (clone LMD932C). FSHD and control hiPSCs clones have been previously described (Badja et al. 2014; Dion et al. 2019). We also derived hiPSCs for another DMD patients using fibroblasts purchased from the Coriell Institite (GM03429, male, Age 6 at sampling; Ex45-50 Del).

Individuals have provided written informed consent for the use of their sample for medical research. The study was done in accordance with the Declaration of Helsinki.

### Generation and culture of hiPSCs

Human iPSCs were generated after nucleofection of primary fibroblast with episomal vectors containing *OCT3*/*4, SOX2, KLF4, C-MYC* and shRNA against p53. hiPSCs colonies were picked about 2 weeks after nucleofection based on Embryonic Stem (ES) cell-like morphology. Colonies were grown and expanded in mTeSR1 medium (Stemcells, Grenoble, France) on BD Matrigel^{™} (BD Biosciences, cat, No. 354277) coated dishes. HiPSCs clones were fully characterized using classical protocols as previously described.

### Cell differentiation

At day 0, clumps of cells were mechanically dissociated and transferred in a new Matrigel-coated dish and cultured in Differentiation Medium (DM; Neurobasal medium supplemented with N2 (1X) B27 (1X), ,NEAA (1X), Glutamax (1X) (life technologies)), ITS-A (1X, final concentration; life technologies, ref 51300044), LDN 193189 (0.5 µM, Sigma, **SML0559)** and CHIR99021 (3 µM, Sigma, 5ml-1046). All reagents are diluted in N2. At day 6, medium is changed with a medium containing DM, LDN 193189, IGF-1 (4 ng/ml, Peprotech, No.100-11) and HGF (10 ng/ml, Peprotech, No.100-39) and β Mercaptoethanol (ThermoFisher, No 31350010). At day 8, HGF is removed and medium is changed with DM supplemented with IGF (4ng/ml, Peprotech, No.100-11), β Mercaptoethanol. At day 12, medium is changed with DM supplemented with IGF (4ng/ml, Peprotech, No.100-11), β Mercaptoethanol and DAPT (10 µM, Tocris). Medium is changed every day until Day 17 where medium is changed with DM and IGF (4ng/ml, Peprotech, No.100-11). In situations where muscle differentiation is delayed or where a poor enrichment in muscle fiber is observed, HGF and Basic Fibroblast Growth Factor (FGF2) can be added to the maintenance medium for 7 additional days.

### RNA extraction, quality control, library preparation and sequencing

Total RNA was extracted using the RNAeasy kit (Qiagen) following manufacturer's instructions. Quality, quantitation and sizing of total RNA was evaluated using the RNA 6000 Pico assay (Agilent Technologies Ref. 5067-1513) on an Agilent 2100 Bioanalyzer system. The RNA integrity number (RIN) was calculated for each sample and only samples with a RIN >9 were kept for further use. Genomic DNA was enriched in protein-coding sequences using the in-solution exome capture SeqCap EZ MedExome Kit (Roche) according to the manufacturer's protocol. Libraries were constructed using 2 µg of total RNA after Qubit quantification. The TruSeq Stranded mRNA Library Preparation Kit High Throughput (Illumina, ref RS-122-2103) was used according to the manufacturer's guidelines. Briefly, PolyA+ containing RNA molecules were purified using polyT oligo-attached magnetic beads. Thermal fragmentation was carried out after two rounds of enrichment for PolyA+ mRNA. cDNA was synthesized using reverse transcriptase (Superscript II) and random primers. This was followed by second strand cDNA synthesis, end repair process, adenylation of 3' ends and ligation of the adapters. The products were then purified and enriched with 15 cycles of PCR to create the cDNA library. Libraries were quantified by qPCR using the KAPA Library Quantification Kit for Illumnia Libraries (Roche, ref. 7960140001). Library profiles were assessed using the DNA High Sensitivity LabChip Kit (Agilent Technologies Ref. 5067-4626) on an Agilent Bioanalyzer 2100. Libraries were sequenced on an Illumina Next Seq 500 NextSeq using cartridge of the NextSeq 500/550 High Output v2 kit (150 cycles) (Illumina FC-404-2002).

### RNA-Seq data processing and differential expression analysis

We assessed fastq sequence data quality using FastQC v0.11.5 (Andrews, 2010) and trimmed the reads to remove adapter sequences and low quality bases using Trimmomatic v0.36 (Maffioletti et al. 2015). The resulting trimmed paired-end reads were aligned using STAR v2.5.3a (Pawlowski et al. 2017) to the GRCh37.p13 human genome release. DEGs were identified using StringTie v1.3.3 and R package edgeR v3.18.0, where raw read counts per gene were normalized using Trimmed Mean of M-values (TMM). Differentially expressed genes were extracted for a fold-change cutoff of 2 and a p value < 0.001. We used the Ensembl human gene IDs identified in the edgeR analyses as input for further analyses. Overrepresentation test analyses were performed using enrichGO from the R package clusterProfiler version 3.10.1. We identified biological processes with an FDR corrected p-value < 0.05 using an universe of 16034 genes (39092 "genes" including variants downsized to 16304 by enrichGO) as reference (D8, D17 and D30 genes with TPM > 0). Results are presented as dotplots with on the *x-axis* the gene count per GO term and on the *y-axis* the identified GO term. Dot size refers to the gene ratio and the color scale represents the adjusted p-value.

Heatmaps were obtained thanks to the pheatmap version 1.0.12 R package using TPM values (https://www.rdocumentation.org/packages/pheatmap). Clustered heatmaps use Ward.D2 as cluster method and Manhattan as distance metric (both on row and column). All heatmaps are scaled by row and the color scale is made from the Z-score. We used gene symbol of identified Ensembl human gene IDs obtained through DESeq2 analyses as input for further analyses.

RNA-seq data and raw count matrix were deposited at the NCBI Gene Expression Omnibus (https://www-ncbi-nlm-nih-gov.gate2.inist.fr/geo/) under the accession GSE142042.

### Gene expression analysis and RT-qPCR

Total RNA was extracted using Trizol (Invitrogen, Cat. No 15596-026). Reverse transcription of 1µg of total RNA was performed using the Superscript III kit and oligo dT following manufacturer's instructions at 42°C for 50 minutes followed by inactivation at 70°C for 15 minutes (Life Technologies). Primers were designed using Primer Blast. PCR amplification was performed on a LightCycler 480 (Roche) using the SYBR green master mix using previously described conditions (Caron et al. 2016). Crossing-threshold (Ct) values were normalized by subtracting the geometric mean of three housekeeping genes (*GAPDH*, *PPIA* and *HPRT1*). All Ct values were corrected by their PCR efficiency, determined by 1:2 or 1:4 cDNA dilution series. Results were treated with the GraphPad prism software for statistical tests (ANOVA, Kruskal-Wallis multiple comparison test; alpha set at 0.05). Only *p*-values less than 0.05 were considered statistically significant.

### Western blot

Proteins were resolved on a 3-8% MOPS-NuPAGE gel (Life Technologies) and transferred on a PVDF membrane (Milipore). Anti-Actin (Millipore MAB1501R) antibodies were used as loading control. Incubation with primary antibodies (MYH8 (1/1000; NOVUS NBP2-41309), MYH3 (1/1000; Santa cruz SC-53091), MYH2 (1/1000; Santa cruz SC-53095), MNX1 (2/1000; Millipore ABN174), DES (1/1000; Abcam AB15200), PAX7 (1/1000 abcam ab187339)) was done overnight at 4°C. After 3 washes in PBS-T, anti-mouse IgG secondary antibody coupled to HRP (ThermoFisher) was incubated for 60 min (1/10 000). Proteins were revealed by chemiluminescence (ECL, Milipore, WBKLS0100) and visualized using a Quantity One Bio-rad camera. Quantified results were treated with the GraphPad prism software for statistical tests (ANOVA, Kruskal-Wallis multiple comparison test; alpha set at 0.05). Only p-values less than 0.05 were considered statistically significant.

### Immunocytochemistry

Cells are fixed in 4% paraformaldehyde, washed with PBS, permeabilized and incubated in blocking buffer containing 3% BSA and 0.8% Triton X100 for 1hour at room temperature. Incubation with primary antibodies (anti-Desmin (1/100; Abcam AB15200), anti-PAX3 (80/1000; DSHB), anti-MYH 2 (1/1000; santa cruz SC-53095), anti-MYH 3 (1/100; santa cruz SC-53091), anti-TTN (96/100; DSHB 9D10), anti- Neurofilament M (NF-M) (1/2000; biolegend PCK-593P), anti-MYH8 (1/100; Novus Biologicals NBP2-41309), anti-Dystrophin (106/1000; DSHB MANDRA 1), anti- MNX1 (1/100; Millipore ABN174), anti-MyoD (1/200; Novus Biologicals NBP1-54153), anti- Islet1 (1/100; Neuromics GT15051)), was done overnight at 4°C in BSA 3%, 0.8% Triton X100. After incubation, cells were washed in PBS and incubated with Alexa Fluor-nonconjugated secondary antibodies (anti-Mouse Alexa Fluor 647 (1/1000; life technologies, A11034), anti-Rabbit Alexa Fluor 555 (1/1000; cell signaling, 44135) or anti-goat Alexa fluor 488 (1/1000; life technologies)) α-Bungarotoxin conjugate (1/400; ThermoFisher B35451) in the presence of 3% BSA; 0.8% Triton X100 for 1 hour. Nuclei were counterstained with DAPI. Images were taken using a confocal imaging system (LSM 800, Zeiss) with a 63X water-immersed lens (63x/1.20 W Korr UV VIS IR, C-Apochromat; Olympus).

### Flow cytometry analysis

Cells are treated with Accutase at 37°C during 10 minutes and rinsed with the N2 medium. For each condition, 1×10⁵ cells were fixed in paraformaldehyde 4% for 20 minutes. After spinning at 1000 rpm for 5 min, cells were rinsed with PBS 1X-BSA 0.2% and then the different antibodies were added (5 µl-25ul of antibody in 100 µl of PBS 1x-BSA 0.2%, ANTI-PAX7 PE (NOVUS NBP2-34706PE), APC-conjugated anti-Pax3 (R&D systems IC2457A)). After 30 min incubation at room temperature, samples were rinsed with PBS 1X and analyzed using an ACCURI C6 flow cytometer on the basis of Forward Scatter (FSC-A) versus side scatter (SSC-A) for the selection of live cells and the elimination of cell aggregates or debris. Analysis of fluorescent population was limited to live cells. Unstained cells and isotype controls were used to determine the background of fluorescence and compensation was determined for individual fluorochromes.

### Imaging and analysis of calcium transients

Calcium transients were measured after addition to the cell culture medium of FLUO-8AM fluorescent Ca²⁺ indicator (AAT Bioquest). Cells were incubated for 30min at 37°c in DM+I medium containing 5µM Fluo8-AM and 0.04% Pluronic acid before imaging on a Fast Imaging Observer system (Axio Observer.Z1/7; Zeiss) with a 10X lens (10x/0.30 M27 DIC I EC Plan-Neofluar; Olympus). Fluorescence was excited at 488nm and emission collected at >509 nm. Images were acquired as time series. Different regions were tracked to record the fluorescent intensity at a 10X magnification during 30 seconds and an interval of 200,0 ms corresponding to 151 frames. Analysis was performed using the Zen pro software (Zeiss). ROI was created for selected fibers then intensity mean for each fiber was exported and Calcium transients were calculated by counting the number of peaks intensity for each graph.

### Cell treatment

For drug treatment, cells were seeded and differentiated in 6 to12-well plates for 45 days. We tested the activity of Mexiletine (M2727, Sigma Aldrich, 10; 100µM diluted in methanol), Carisoprodol (C8759, Sigma Aldrich, 0.5µM; 1µM diluted in ethanol), Salbutamol (S8260, Sigma Aldrich, 1µM; 10µM, diliuted in methanol) and L-Glutamic acid potassium salt monohydrate (G1501, Sigma Aldrich, 2 µM; 20 µM diluted in H₂O) added at 0.1% in the culture medium on fiber contractions. Cell contractions were captured as described for Calcium handling measurements.

### Electron microscopy

After 5 minutes of wash with 0.1M sodium cacodylate buffer, cells were directly fixed in solution with glutaraldehyde 2.5% in 0.1M sodium cacodylate buffer during 1h at room temperature, then 3 times washed for 10 minutes with 0.1M cacodylate buffer. Cells were post-fixed with 2% osmium tetroxyde's (in 0.1M cacodylate buffer) steam during 45 minutes, then they were washed again 3 times for 15 minutes with distilled water. Contrast with 1% Uranyl acetate in water was performed overnight at 4°C. After 3 washes with distilled water, progressive deshydratation is required with 50% to 100% Ethanol baths before start the embedding in Epoxy resin (EPON 812) from 33% to 100% resin, then overnight polymerisation at 55°C. Wells are prepared to make apico-basal axis blocks to cut. Ultrathin 60nm sections were obtained using Ultracut-E ultramicrotome (Reichert-Jung, Southbridge, Massachusetts, USA). Pictures were obtained using JEM-1400 transmission electron microscope (JEOL, Tokyo, JAPAN) at 80kV with Megaview III Camera (SIS Imaging, Münster, Germany).

### Results

### Example 1: Experimental procedure and cell differentiation overview

After mechanical dissociation, small clumps of hiPSCs are collected and plated on Matrigel-coated dishes, in the presence of Thiazovivin (Figure 1) in differentiation medium (DM) supplemented with ITS-A, LDN193189 (LDN), a potent BMP pathway inhibitor and CHIR99021, a GSK3 inhibitor. Cells are maintained for 6 days in this medium with daily medium change. On the 7^{th} day (D7), cells are grown in DM+LDN supplemented with Insulin Growth Factor 1 (IGF1) and Hepatocyte Growth Factor (HGF) for 24 hrs to favor muscle and neuron progenitors differentiation. LDN and HGF are then removed and cells are maintained in DM+IGF1 for four more days (until day 12) during which cells proliferate. Between days 10-12, cells can be collected by dissociation with accutase for freezing, storage, thawing and re-plating. At day 12 (D12), DAPT, a γ secretase and Notch pathway inhibitor is added to the medium to induce neuronal differentiation. From D17 onwards, cells in DM+IGF1 form large patches of elongated contractile fibers clearly visible by bright field imaging. First contractions become visible 2 to 4 days after DAPT removal (days 19-20), with axons visible by bright field imaging (D17).

### Example 2: hiPSC differentiation leads to the progressive enrichment in skeletal muscle progenitors followed by formation of myofibers.

We analyzed expression of *PAX3* and *PAX7* that characterize muscle progenitors by immunostaining. PAX3 was detected as early as D6 in the vast majority of cells and remained visible at D8. At D12, cells express *PAX7,* a marker of muscle satellite cells, indicating commitment towards the skeletal muscle lineage. Upon medium change (DM + IGF1, DAPT), we observed a progressive change in cell shape and appearance of elongated cells, positive for the sarcomeric Desmin marker. Using flow cytometry; we detected approximately 12% of PAX3-positive (PAX3+) cells and 2% PAX7+ cells at day 8 and 2-5% of PAX7+ cells from D21 onwards (data not shown) indicating that our experimental condition maintains a pool of satellite-like cells expressing *PAX7* suggesting self-regenerative capabilities.

### Example 3: hiPSC-derived myotubes show complete sarcomeric differentiation and highly organized myofibrillar pattern.

To further characterize cells at the end of the differentiation protocol, muscle fibers were analyzed at day 45 post-differentiation using anti-Titin (TTN), a sarcomeric protein that links the Z disk and M line and anti-Desmin (DES) responsible for connecting myofibrils to each other and to the plasma membrane. We observed cell fusion and formation of multinucleated fibers expressing Titin, and Desmin. By transmission electron microscopy (TEM), myofibers display a well-organized sarcomeric structure with clearly visible A-band of Myosin filaments, I-bands of Actin filaments, M- and Z-lines (Figure 2, panel 2 and 4, "Z" and "M"), all hallmarks of mature sarcomeres. Mitochondria (Figure 2, panel 3, "Mt") are large and display well-organized cristae indicative of a high metabolic activity, consistent with the continuous contraction of cells. In addition, myofibers show multiple nuclei at the periphery of the fibers (Figure 2, panel 1, "N"), a marker of differentiation and maturation.

### Example 3: Myotubes and motor neurons in close proximity connect with each other to form functional neuromuscular junctions.

To address whether contractions starting at day 19-21 occur spontaneously or are associated with nervous stimuli, presence of acetylcholine receptors (AChR) and neuromuscular junction (NMJ) were visualized by staining with Alexa Fluor 555 α Bungarotoxin (αBtx). We observed several AChR at the surface of myofibers suggesting the presence of undifferentiated acetylcholine receptors clusters with motor neuron axons that extend towards these αBtx clusters. To functionally characterize these myofibers, we analyzed intracellular Calcium (Ca²⁺) release at D30, 4 (D120) or 5 (D150) months post differentiation by adding Fluor8 to fresh medium 30 minutes prior to recording of spontaneous Ca²⁺ transients per minute and per fiber (Figure 3A). We did not observe any difference in Calcium release between early and late time points, indicating that muscle functionality is maintained even for long periods in culture. Furthermore, consistent with the presence of AchR and motor neurons, fiber contractions are irreversibly blocked by addition of αBtx and Tetrododotxin (TTX) (Figure 3B) advocating for the presence of functional NMJs.

### Example 4: Activation of genes involved in muscle contraction occurs between D17 and D30

To follow the gene expression profiles at key steps of the differentiation process, we performed an RNA Seq transcriptome analysis in two different control cells at D8, D17 and D30, corresponding to the main medium changes. As illustrated by volcano plots, the number of differentially expressed genes is high between D8 and D17 and decreases between D17 and D30 (Figure 4).

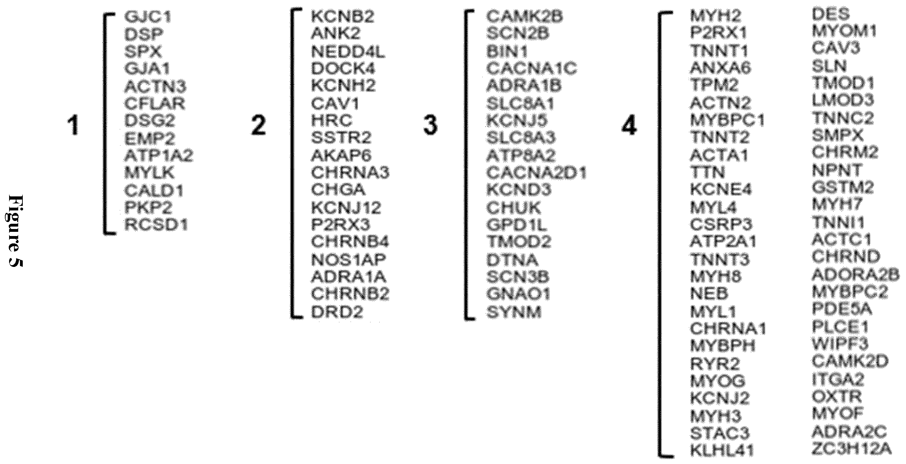

By selecting genes with Log2 fold change (Log2FC) of 2 and an adjusted *p* value (*p*adj) < 0.05 and comparing genes differentially expressed (DEGs) between D8-D17 and D17-D30 corresponding to GO terms related to "contraction", we obtained a first list of 101 DEGs subdivided into 4 groups (Figure 5). The first group (13 genes), activated at D8 and silenced at D17-D30 corresponds to genes involved in "myocytes adhesion and cell communication", "muscle system process" or "cardiac conduction". The second group (18 genes), transiently activated at D17 and silenced at D30 corresponds to genes involved in smooth muscle and cardiac muscle contraction. The third group (18 genes), activated at D17 and decreased at D30 corresponds to genes involved in "Calcium ion transmembrane transport via high voltage-gated Calcium channel" "cardiac action potential and membrane depolarization", suggesting as observed in group 2, the transient activation of genes involved in cardiac contraction. Consistent with the first contractions at D19-21, the fourth group (52 genes), specifically activated at D30 corresponds to "muscle filament sliding' (21 genes, *p*adj: 7.75e⁻⁴²), "relaxation of skeletal muscle", "detection of muscle stretch", "regulation of twitch skeletal muscle contraction" indicating a progressive enrichment in pathways corresponding to skeletal muscle function.

### Example 5: Transcriptomic profiling shows a 2-phase process with induction of neuronal differentiation followed by induction of the myogenic program.

Comparison of DEGs between the three datasets (D8, D17, D30) revealed a list of 2229 DEGs between D8 and D17 and 281 genes between D17 and D30, with an overlap of 302 genes common to the three time points. For this group, the fifteen most significant associated GO terms correspond to skeletal muscle function and development or axonogenesis. Analysis of biological pathways significantly overrepresented between D8-D17 and D17-D30, revealed two phases in the differentiation process. Between D8-D17, enriched pathways correspond to axon development and synapse formation but also cell cycle regulation, consistent with final differentiation and cell cycle exit . In the second phase (D17- D30), we observed an enrichment in motor neuron networks with a marked increase in *TUBB3,* a class-III β-Tubulin restricted to neurons expressed in dorsal root ganglion and required for axon outgrowth (Luduena et al. 1993) .

### Example 6: Myogenic and neuronal specification and long-term persistence of PAX7+ cells.

Between D17 and D30, Notch pathway inhibition and activation of neuronal differentiation facilitates skeletal muscle differentiation as evidenced by progressive activation of myogenesis and overrepresentation of genes specific to skeletal muscle differentiation and development.

At the individual gene level, expression of embryonic/fetal Myosins is followed by the progressive increase of adult isoforms (MYH2 and MYH7). We also noticed expression of *NCAM1* encoding the CD56 cell surface marker between D8 and D17 (Log2FC: -3.92,*p*adj: 3.75e⁻⁸) and a steady expression between D17 and D30 (Log2FC: 1.077) indicating a stable proportion of precursor cells over time. From D8 to D17, we observed an increased in *MEOX1* expression, activated in pre-somitic mesoderm and required for sclerotome and somitic development (Log2FC: 3.56, *p*adj: 8.49e⁻⁶) and an increase in *PAX3* (Log2FC: 3.03, *p*adj: 6.93e⁻¹⁴). *PAX7* expression significantly increases between D17 and D30 (Log2FC: -2.46, *p*adj: 4.56e⁻⁸), consistent with immunostaining data (see example 2) and replacement of PAX3+ muscle/neuronal precursors to PAX7+ muscle progenitors. Regarding myogenic markers, *MYF5* is expressed at a low level at the different time points while *MYOD1* strongly increases (Log2FC: -3.56, *p*adj: 3.42e⁻⁵) between D17-D30. Late differentiation markers, *MYOG* and *MYF6* are poorly expressed between D8 and D17 and progressively increase between D17 and D30 (Log2FC: - 2.89, *p*adj: 5.27e⁻⁶; Log2FC: -1.92, *p*adj: 0.08, respectively) consistent with their role at late differentiation stages and myotube/myofiber formation.

DEGs for muscle-associated GO terms provided a list of 21 genes expressed in other mesoderm-derived tissues which expression decreases between D17 and D30. The list of upregulated genes (111 genes) are involved in skeletal muscle function and include muscle-specific myosin heavy chains (MHC) such as embryonic/fetal MYH8, MYH3 but also adult MYH7 and MYH2 isoforms as well as Troponin T such as *TNNC2, TNNT3* or *TNNT2* expressed in fast type skeletal muscles and *TNNC1,* expressed in slow type skeletal muscle. We also detected increased expression of two recently discovered genes that encode muscle specific membrane proteins required for fusion and myotube formation, *MYMK* (Myomaker) and *MYMX* (encoding Minion, Myomerger or Myomixer) (Zhang et al. 2017; Millay et al. 2013). Myomaker required for membrane hemifusion is activated at early differentiation stages (Log2FC: -9.84; *p*adj: 7.63E⁻¹⁰ between D8 and D30) while Myomixer required for fusion completion is activated at later stage (Log2FC: -3.7; padj: 8.42E⁻⁷ between D17 and D30), consistent with its action in fusion of Myomaker-positive cells (Zhang et al. 2017).

In addition, overrepresentation tests revealed a strong enrichment in genes encoding components of the extracellular matrix (ECM) between D17 and D30, , in which we noticed different Laminins, members of the Collagen family (*p*adj: 1.26e⁻¹⁰) and activation of the TGFβ pathway. ECM synthesis likely contributes to organization of the innervated muscle tissue, its long-term maintenance and mechanical properties without massive cell detachment and cell death as usually reported for primary myoblasts or hiPSC-derived myoblasts after the first contractions (Gillies et al. 2011).

### Example 7: Months-long persistence of muscle and motoneurons signature.

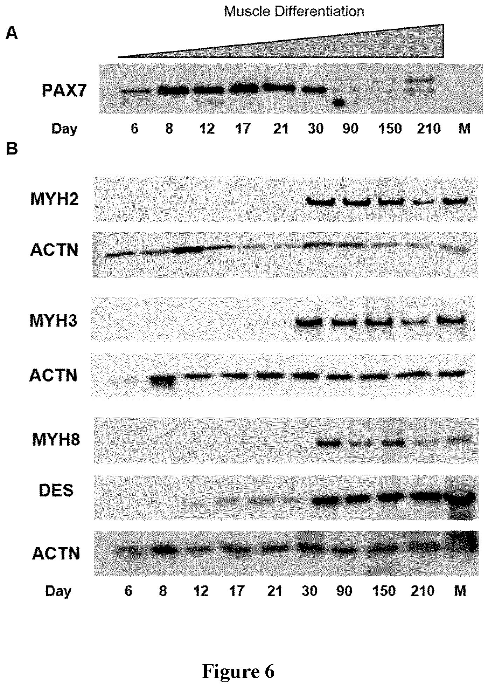

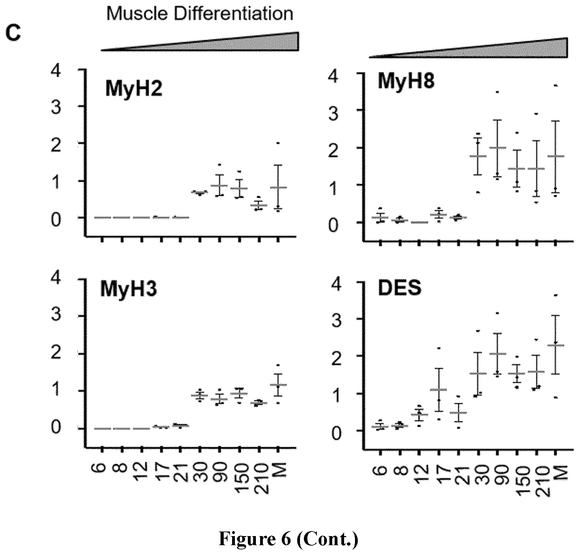

For a selected number of genes, RNA seq data were confirmed by RT-qPCR from D6 to D30 and at late time points (3 months, 5 months, 7 months) post differentiation. Expression of the adult *MYH2* isoform was detectable as early as D8 and remained steady with time (Figure 6). The same kinetics is observed for Desmin (*DES*), Titin *(TTN),* Sarcoglycan Gamma Sarcolemmal protein *(SGCG), RYR1* encoding the Ryanodine Receptor involved in Calcium release in the sarcoplasmic reticulum and myogenic transcription factors (*MYOD1*, *MYF5, MYOG*, MYF6. Expression of *PAX3* is stable from D6-D21 then decreases at D30, while expression of *PAX7* progressively increases and remains stable over time suggesting that precursor cells able to regenerate the culture persist over time. Kinetics of *PAX7, Desmin* and *MYH2, 3* and *8* expression were confirmed by western blotting (Figure 6A), all of which remained detectable for up to 7 months post-differentiation (Figure 6A-C).

Expression of motor neurons specific markers such as the HB9 homeobox protein (*MNX1*) is higher at early differentiation stages and decreases at D30 while *ISLET1* (*ISL LIM homeobox 1*) expression increases upon DAPT induction and remains stable. Consistent with fiber contractions, presence of NMJs and inhibitory action of αBTX, expression of Choline-O-Acetyltransferase (*ChAT*) specific of cholinergic neurons and Agrin (*AGRN*) increases from D17 onwards and remains. Together with expression of the Myelin Basic Protein (MBP)), we also detected cells positive for S100β that colocalize with αBTX-stained NMJs indicating the presence of Schwann cells. In agreement with formation of functional NMJs, TEM revealed basal lamina invaginations and the presence of synaptic cleft at the surface of muscle (Figure7). Thus, self-organization between muscle cells, motors neurons and neural crest-derived cells permits production of highly organized innervated muscle fibers.

### Example 8: Response to pharmaceutical drugs.

To evaluate the potential application of hiPSC-derived innervated myofibers as a preclinical model for drug testing, we tested the response to different classes of pharmaceutical agents by real time video microscopy and Calcium handling analysis. Cells at D45 were treated for different durations with different concentrations of each molecule at (Figure 8A-D). We did not observe any difference in Calcium release after addition of 2µM Glutamate but an increased activity 20 to 30 minutes for a 20 µM concentration, consistent with expression of Glutamate receptors and participation of Glutamate in modulating Acetylcholine transmission (Figure 8A).

We then tested the action of three muscle relaxants, Salbutamol, a β2 adreno receptor agonist with a potent relaxant property, Carisoprodol, that modulates γ-Aminobutyric acid type A receptor (GABA_{A}Rs) post-synaptic neurotransmission and Mexiletine, a Sodium channel blocker (Naᵥ 1.4) used to reduce muscle stiffness, tiredness and weakness, in particular in DM (Logigian et al. 2010) . Salbutamol decreases Ca²⁺ handling as early as 4 hours (hrs) after 1 µM and 10 µM drug addition to the medium with recovery to the basal activity after 24 hrs (Figure 9B). The effect of Carisoprodol that modulates post-synaptic neurotransmission and reduces muscle contraction 3 to 4 hrs after addition of 0.5 to 1 mM drug, with total recovery after 24 hrs (Figure 8C). As expected for its activity to block Sodium channel and inhibit Na⁺/Ca²⁺ exchange-dependent Ca²⁺ overload, we observed a decrease in Calcium handling 2 hrs after addition of 10 µM Mexiletine and a sharp decrease 1 to 2 hrs when 100 µM drug was added followed by a progressive but partial recovery between 3 to 24 hrs post-treatment (Figure 8D). Altogether, functional response to these different drugs highlights the value of our "innervated muscle in a dish" model for drug screening and therapeutic development.

### Example 9: hiPSC-derived muscle cells for modeling neuromuscular disorders.

To evaluate the reproducibility and robustness of our protocol, we applied our methodology to the most frequent muscular dystrophies, Duchenne Muscular Dystrophy (DMD, OMIM 310200), Myotonic Dystrophy (DM1, OMIM 160900), type 2 Facio Scapulo Humeral Dystrophy (FSHD2, OMIM 158901) and Limb Girdle Muscular Dystrophy 2A (LGMD2A, or LGMDR1, OMIM 253600). Using antibodies against Desmin and Titin, we observed the formation of multinucleated millimeter-long aligned myofibers and a mature fiber striated pattern at D30 post-differentiation. We also observed immature AChR clusters at the surface of the fibers after α BTX staining. Compared to controls, fiber section size is reduced in DMD cells, more variable and slightly increased in FSHD2 cells (Figure 9). Expression of the different genes validated in control cells was evaluated by RT-qPCR from D6 to D30. Expression of embryonic and fetal MyHCs is comparable in the different contexts while expression of the adult isoform (*MYH2*) is more variable suggesting delayed muscle maturation in diseases. Expression of *Desmin, Titin* and *PAX7* is comparable between conditions. At the protein level, MYHCs are detectable earlier in DM1 but at a lower level compared to controls and follows a similar kinetics as in controls for the other pathologies.

In addition our preliminary analysis of the transcriptomic profile of genes mutated in muscle diseases and tested on whole exome sequencing gene panels, highlights the value of our model to reveal signatures associated with genetic neuromuscular diseases.

### Example 10 : Terminal differentiation of hiPSCs derived from patients suffering from muscular dystrophies.

In the four pathologies, TEM revealed a full maturation of hiPSC-derived muscle fibers with visible Z lines structures (Figure 10). However, we observed an increased proportion of discontinuous and tortuous architecture with interrupted Z lines suggesting defects in sarcolemmal anchoring as reported (Reilich et al. 2010; Ludatscher et al. 1978). In DMD, the presence of vacuole-like areas between fibers, strikingly resembles to what was reported *in vivo* Nadaj-Pakleza et al. 2011 and muscle fiber degeneration (Figure 10). In LGMD2A, we observed a patchy pattern of small disorganized fibers with electron-dense inclusion between fibers as reported (Kramerova et al. 2004; Vainzof et al. 2003). Visualization of these structural abnormalities further confirms that our protocol can be reliably used to model neuromuscular pathologies.

### Example 11: Monitoring of Calcium handling in neuromuscular diseases.

Having shown that co-differentiation of neurons and myotubes permits formation of NMJs at the surface of the fibers in healthy and diseased context, we next investigated muscle fibers functionality by measuring Calcium transient at D30. We observed a significant decrease in Calcium handling amplitude in DMD cells (*p*<0.0001) compared to controls but an increase in the other pathologies (DM1, FSHD and LGMD; *p*<0.0001, Figure 11) attesting muscle fibers functionality for the different diseases and providing a reliable cellular model and potential readout for functional testing.

### REFERENCES

K. Takahashi and S. Yamanaka, Induction of pluripotent stem cells from mouse embryonic and adult fibroblast cultures by defined factors. Cell 2006; 126: 663-676 (Aug, 2006).
K. Takahashi et al., Induction of pluripotent stem cells from adult human fibroblasts by defined factors. Cell 2007; 131: 861-872 (Nov, 2007).
I. H. Park et al., Reprogramming of human somatic cells to pluripotency with defined factors. Nature 2008; 451: 141-146 (Jan, 2008).
W. S. Hu et al., Large-sale mammalian cell culture. Curr Opin Biotechnol 8(2):148-53 (Apr, 1997).
K. Kitano, Serum-free media. Biotechnology 17: 73 (1991).
R. E. Spier, Large-scale mammalian cell culture: methods, applications and products. Curr Opin Biotechnol 2(3): 375-9 (Jun, 1991).
E. J. Robertson, Teratocarcinomas and embryonic stem cells: A practical approach. ed., IRL Press Ltd (1987).
P. M. Wasserman et al., Guide to Techniques in Mouse Development. eds., Academic Press (1993).
N. J. Totowa, Embryonic Stem Cells: Methods and Protocols. Kursad Turksen, ed., Humana Press (2001).
M. V. Wiles, Embryonic Stem Cell Differentiation in Vitro. Meth. Enzymol. 225: 900 (1993).
P. D. Rathjen et al., Properties and uses of Embryonic Stem Cells: Prospects for Application to Human Biology and Gene Therapy. Reprod Fertil Dev. 10(1):31-47 (1993).
E. J. Robertson, Derivation and maintenance of embryonic stem cell cultures. Methods Mol Biol. 75:173-84 (1997).
M. L. Roach and J. D. McNeish, Methods for the isolation and maintenance of murine embryonic stem cells. Methods Mol Biol. 185:1-16 (2002).
Y. E Antebi et al., Combinatorial signal perception in the BMP pathway. Cell 170(6): 1184-1196 (Sep, 2017).
M. J. Borok et al., Bu-M-P-ing Iron: How BMP Signaling Regulates Muscle Growth and Regeneration. J Dev Biol 8(1): 4 (Mar, 2020).
C. Badja et al., Efficient and cost-effective generation of mature neurons from human induced pluripotent stem cells. Stem Cells Transl Med 3(12): 1467-72 (Dec, 2014).
C. Dion et al. SMCHD1 is involved in de novo methylation of the DUX4-encoding D4Z4 macrosatellite. Nucleic Acids Res. 8;47(6): 2822-2839 (Apr, 2019).
T. Barberi et al., Derivation of engraftable skeletal myoblasts from human embryonic stem cells. Nat Med 13, 642 (May, 2007).
Q. Zhang et al., The microprotein Minion controls cell fusion and muscle formation. Nat Commun 8, 15664 (Jun 1, 2017).
D. P. Millay et al., Myomaker is a membrane activator of myoblast fusion and muscle formation. Nature 499, 301 (Jul 18, 2013).
A. R. Gillies, R. L. Lieber, Structure and function of the skeletal muscle extracellular matrix. Muscle Nerve 44, 318 (Sep, 2011).
E. L. Logigian et al., Mexiletine is an effective antimyotonia treatment in myotonic dystrophy type 1. Neurology 74, 1441 (May 4, 2010).
P. Reilich et al., Facioscapulohumeral muscular dystrophy presenting with unusual phenotypes and atypical morphological features of vacuolar myopathy. J Neurol 257, 1108 (Jul, 2010).
R. M. Ludatscher, H. Kerner, S. Amikam, B. Gellei, Myotonia dystrophica with heart involvement: an electron microscopic study of skeletal, cardiac, and smooth muscle. J Clin Pathol 31, 1057 (Nov, 1978).
A. Nadaj-Pakleza et al., Muscle pathology in myotonic dystrophy: light and electron microscopic investigation in eighteen patients. Folia Morphol (Warsz) 70, 121 (May, 2011).
I. Kramerova, E. Kudryashova, J. G. Tidball, M. J. Spencer, Null mutation of calpain 3 (p94) in mice causes abnormal sarcomere formation in vivo and in vitro. Hum Mol Genet 13, 1373 (Jul 1, 2004).
M. Vainzof, F. de Paula, A. M. Tsanaclis, M. Zatz, The effect of calpain 3 deficiency on the pattern of muscle degeneration in the earliest stages of LGMD2A. J Clin Pathol 56, 624 (Aug, 2003).

## Claims

1. A method for the production, from Pluripotent Stem Cells (PSCs), of functional skeletal muscle fibers innervated by motoneurons, comprising the step of:
a) culturing PSCs in a culture medium comprising a Bone Morphogenic Proteins (BMP) pathway inhibitor and a Wingless and Int-1 (Wnt) pathway inhibitor during 1 to 10 days;
b) switching the cells to a culture medium comprising a BMP pathway inhibitor, Insulin Growth Factor 1 (IGF-1) and Hepatocyte Growth Factor (HGF), and culturing the cells during 1 to 3 days;
c) switching the cells to a culture medium containing IGF1 and lacking HGF and a BMP pathway inhibitor, and culturing the cells during 1 to 8 days;
d) switching the cells to a culture medium containing IGF1 and a γ-secretase and Notch pathway inhibitor, and culturing the cells during 1 to 10 days; and
e) switching the cells to a culture medium suitable to maintain the thus produced functional skeletal muscle fibers innervated by motoneurons.

2. The method according to claim 1, wherein the Wnt pathway inhibitor of step a) is an indirect GSK3 inhibitor or a direct GSK3 inhibitor, such as a direct GSK3 inhibitor selected in the group consisting of CHIR99021, lithium, 6-bromoindirubin-3-oxime, SB216763, SB415286, CHIR98014, bikinin, TDZD-8, LY2090314 and (2'Z) indirubin.

3. The method according to claim 2, wherein the Wnt pathway inhibitor of step a) is CHIR99021.

4. The method according to any one of claims 1 to 3, wherein the culture medium of step a) further comprises a Rho-associated protein kinase (ROCK) pathway inhibitor, such as thiazovivin Rho kinase inhibitor IV, Fasudil, GSK429286A, or Y-27632.

5. The method according to any one of the claims 1 to 4, wherein the BMP pathway inhibitor of steps a) and b) is selected in the group consisting of Bone morphogenetic protein receptor type IB (Bmpr1b, or ALK6) inhibitors, Activin A receptor type I (ACVR1, or ALK2) inhibitors, Activin receptor-Like Kinase 1 (ALK1) inhibitors, Bone morphogenetic protein receptor type II (Bmpr2) inhibitors, Activin receptor type IIA (Acvr2a) inhibitors, and Activin receptor type IIB (Acvr2b) inhibitors; said BMP pathway inhibitor being in particular an inhibitor of both ALK2 and ALK3 receptors.

6. The method according to claim 5, wherein the BMP pathway inhibitor of step a) and b) is LDN193189 (LDN).

7. The method according to any one of the claims 1 to 6, wherein the γ-secretase and Notch pathway inhibitor of step d) is selected in the group consisting of N-[N-(3,5-Difluorophenacetyl)-L-alanyl]-S-phenylglycine t-butyl ester (DAPT), compound E, tarenflurbil and dibenzazepine.

8. The method according to claim 7 wherein the γ-secretase and Notch pathway inhibitor of step d) is DATP.

9. The method according to the any one of claims 1 to 8, wherein the duration of step a) is about 6 days, and/or the duration of step b) is about 1 day, and/or the duration of step c) is about 4 days, and/or the duration of step d) is about 5 days.

10. The method according to any one of the claims 1 to 9, wherein the PSCs of step a) are induced PSCs (iPSCs).

11. The method according to claim 10, wherein iPSCs of step a) are derived from fibroblast, in particular from human fibroblasts.

12. The method according to any one of claims 10 or 11, wherein iPSCs of step a) are derived from cells of a patient with a neuromuscular disease or a muscular disorder.

13. The method according to claim 12, wherein the neuromuscular disease is a muscular dystrophy selected in the group consisting of Duchenne Muscular Dystrophy (DMD), Myotonic Dystrophy (MD), Facio-Scapulo-Humeral Dystrophy (FSHD) and type 2A Limb-Girdle Muscular Dystrophy (LGMD2A).

14. The method according to any one of claim 1 to 13, wherein the culture medium of steps a) to e) is a serum-free medium.

15. The method according to any one of claims 1 to 14, wherein the cells are cultured in step a) to e) on a surface coated with laminin, type IV collagen and proteoglycans.

## Patentansprüche

1. Verfahren zur Herstellung von funktionellen Skelettmuskelfasern, die von Motoneuronen innerviert werden, aus pluripotenten Stammzellen (PSCs), umfassend den Schritt:
a) Kultivierung von PSCs in einem Kulturmedium, das einen Inhibitor des Bone Morphogenic Proteins (BMP)-Stoffwechselwegs und einen Inhibitor des Wingless- und Int-1 (Wnt)-Stoffwechselwegs enthält, während 1 bis 10 Tagen;
b) Umstellung der Zellen auf ein Kulturmedium, das einen BMP-Stoffwechselweg-Inhibitor, Insulin-Wachstumsfaktor 1 (IGF-1) und Hepatozyten-Wachstumsfaktor (HGF) enthält, und Kultivierung der Zellen während 1 bis 3 Tagen;
c) Umstellung der Zellen auf ein Kulturmedium, das IGF1 enthält und in dem HGF und ein BMP-Weg-Inhibitor fehlen, und Kultivierung der Zellen während 1 bis 8 Tagen;
d) Umstellen der Zellen auf ein Kulturmedium, das IGF1 und einen y-Sekretase- und Notch-Signalweg-Inhibitor enthält, und Kultivieren der Zellen während 1 bis 10 Tagen; und
e) Umstellung der Zellen auf ein Kulturmedium, das geeignet ist, die so erzeugten funktionellen Skelettmuskelfasern, die von Motoneuronen innerviert werden, zu erhalten.

2. Das Verfahren nach Anspruch 1, wobei der Wnt-Signalweg-Inhibitor von Schritt a) ein indirekter GSK3-Inhibitor oder ein direkter GSK3-Inhibitor ist, wie ein direkter GSK3-Inhibitor, ausgewählt aus der Gruppe bestehend aus CHIR99021, Lithium, 6-Bromindirubin-3-oxim, SB216763, SB415286, CHIR98014, Bikinin, TDZD-8, LY2090314 und (2'Z)-Indirubin.

3. Das Verfahren nach Anspruch 2, wobei der Wnt-Signalweg-Inhibitor von Schritt a) CHIR9902I ist.

4. Das Verfahren nach einem der Ansprüche 1 bis 3, wobei das Kulturmedium von Schritt a) ferner einen Rho-assoziierten Proteinkinase (ROCK)-Signalweg-Inhibitor enthält, wie Thiazovivin Rho-Kinase-Inhibitor IV, Fasudil, GSK429286A oder Y-27632.

5. Das Verfahren nach einem der Ansprüche 1 bis 4, wobei der BMP-Signalweg-Inhibitor der Schritte a) und b) ausgewählt ist aus der Gruppe bestehend aus Knochenmorphogenetischen Proteinrezeptor Typ IB (Bmprlb oder ALK6) Inhibitoren, Activin-A-Rezeptor Typ I (ACVR1, oder ALK2) Inhibitoren, Activin Rezeptor-Like Kinase 1 (ALK1) Inhibitoren, Knochenmorphogenetischen Protein Rezeptor Typ II (Bmpr2) Inhibitoren, Activin-Rezeptor Typ IIA (Acvr2a) Inhibitoren, und Activin-Rezeptor Typ IIB (Acvr2b) Inhibitoren; wobei der Inhibitor des BMP-Wegs insbesondere ein Inhibitor der beiden Rezeptoren ALK2 und ALK3 ist.

6. Das Verfahren nach Anspruch 5, wobei der BMP- Signalweg -Inhibitor von Schritt a) und b) LDN193189 (LDN) ist.

7. Das Verfahren nach einem der Ansprüche 1 bis 6, wobei der y-Sekretase- und Notch-Signalweg-Inhibitor aus Schritt d) ausgewählt ist aus der Gruppe bestehend aus N-[N-(3,5-Difluorphenacetyl)-L-alanyl]-S-phenylglycin-t-butylester (DAPT), Verbindung E, Tarenflurbil und Dibenzazepin.

8. Das Verfahren nach Anspruch 7, wobei der y-Sekretase- und Notch- Signalweg -Inhibitor von Schritt d) DATP ist.

9. Das Verfahren nach einem der Ansprüche 1 bis 8, wobei die Dauer von Schritt a) etwa 6 Tage beträgt, und/oder die Dauer von Schritt b) etwa 1 Tag beträgt, und/oder die Dauer von Schritt c) etwa 4 Tage beträgt, und/oder die Dauer von Schritt d) etwa 5 Tage beträgt.

10. Das Verfahren nach einem der Ansprüche 1 bis 9, wobei die PSCs aus Schritt a) induzierte PSCs (iPSCs) sind.

11. Das Verfahren nach Anspruch 10, wobei die iPSCs aus Schritt a) von Fibroblasten, insbesondere von menschlichen Fibroblasten, stammen.

12. Das Verfahren nach einem der Ansprüche 10 oder 11, wobei die iPSCs aus Schritt a) von Zellen eines Patienten mit einer neuromuskulären Krankheit oder einer Muskelerkrankung stammen.

13. Das Verfahren nach Anspruch 12, wobei die neuromuskuläre Erkrankung eine Muskeldystrophie ist, ausgewählt ist aus der Gruppe bestehend aus Duchenne-Muskeldystrophie (DMD), Myotonischer Dystrophie (MD), Facio-Scapulo-Humeral-Dystrophie (FSHD) und Gliedergürtel-Muskeldystrophie Typ 2A (LGMD2A).

14. Das Verfahren nach einem der Ansprüche 1 bis 13, wobei das Kulturmedium der Schritte a) bis e) ein serumfreies Medium ist.

15. Das Verfahren nach einem der Ansprüche 1 bis 14, wobei die Zellen in Schritt a) bis e) auf einer mit Laminin, Typ-IV-Kollagen und Proteoglykanen beschichteten Oberfläche kultiviert werden.

## Revendications

1. Méthode pour la production, à partir de cellules souches pluripotentes (PSCs), de fibres musculaires squelettiques fonctionnelles innervées par des neurones moteurs, comprenant les étapes de :
a) culture de PSCs dans un milieu de culture comprenant un inhibiteur de la voie des protéines osseuses morphogénétiques (BMP) et un inhibiteur de la voie Wingless et Int-1 (Wnt) pendant 1 à 10 jours ;
b) basculement des cellules dans un milieu de culture comprenant un inhibiteur de la voie BMP, le facteur de croissance insulinique 1 (IGF-1) et le facteur de croissance des hépatocytes (HGF), et culture des cellules pendant 1 à 3 jours ;
c) basculement des cellules dans un milieu de culture contenant IGF1 et dépourvu de HGF et d'un inhibiteur de la voie BMP, et culture des cellules pendant 1 à 8 jours ;
d) basculement des cellules dans un milieu de culture contenant IGF1 et un inhibiteur de la voie de γ-sécrétase et Notch, et culture des cellules pendant 1 à 10 jours ; et
e) basculement des cellules dans un milieu de culture convenant pour maintenir les fibres musculaires squelettiques fonctionnelles innervées par des neurones moteurs ainsi produites.

2. Méthode selon la revendication 1, dans laquelle l'inhibiteur de la voie Wnt dans l'étape a) est un inhibiteur de GSK3 indirect ou un inhibiteur de GSK3 direct, tel qu'un inhibiteur de GSK3 direct choisi dans le groupe constitué par CHIR99021, le lithium, la 6-bromoindirubine-3-oxime, SB216763, SB415286, CHIR98014, la bikinine, TDZD-8, LY2090314 et la (2'Z) indirubine.

3. Méthode selon la revendication 2, dans laquelle l'inhibiteur de la voie Wnt dans l'étape a) est CHIR99021.

4. Méthode selon l'une quelconque des revendications 1 à 3, dans laquelle le milieu de culture dans l'étape a) comprend en outre un inhibiteur de la voie de protéine kinase associée à Rho (ROCK) tel que la thiazovivine, l'inhibiteur IV de Rho kinase, le fasudil, GSK429286A, ou Y-27632.

5. Méthode selon l'une quelconque des revendications 1 à 4, dans laquelle l'inhibiteur de la voie BMP dans les étapes a) et b) est choisi dans le groupe constitué par les inhibiteurs de récepteur de protéine osseuse morphogénétique de type IB (Bmpr1b ou ALK6), les inhibiteurs de récepteur d'activine A de type 1 (ACVR1 ou ALK2), les inhibiteurs de kinase 1 semblable au récepteur d'activine (ALK1), les inhibiteurs de récepteur de protéine osseuse morphogénétique de type II (Bmpr2), les inhibiteurs de récepteur d'activine de type IIA (Acvr2a), et les inhibiteurs de récepteur d'activine de type IIB (Acvr2b) ; ledit inhibiteur de la voie BMP étant en particulier un inhibiteur des deux récepteurs ALK2 et ALK3.

6. Méthode selon la revendication 5, dans laquelle l'inhibiteur de la voie BMP dans les étapes a) et b) est LDN193189 (LDN).

7. Méthode selon l'une quelconque des revendications 1 à 6, dans laquelle l'inhibiteur de la voie de γ-sécrétase et Notch dans l'étape d) est choisi dans le groupe constitué par l'ester t-butylique de N-[N-(3,5-difluorophénacétyl)-L-alanyl]-S-phénylglycine (DAPT), le composé E, le tarenflurbil et la dibenzazépine.

8. Méthode selon la revendication 7, dans laquelle l'inhibiteur de la voie de γ-sécrétase et Notch dans l'étape d) est le DATP.

9. Méthode selon l'une quelconque des revendications 1 à 8, dans laquelle la durée de l'étape a) est d'environ 6 jours, et/ou la durée de l'étape b) est d'environ 1 jour, et/ou la durée de l'étape c) est d'environ 4 jours, et/ou la durée de l'étape d) est d'environ 5 jours.

10. Méthode selon l'une quelconque des revendications 1 à 9, dans laquelle les PSCs dans l'étape a) sont des PSCs induites (iPSCs).

11. Méthode selon la revendication 10, dans laquelle les iPSCs dans l'étape a) dérivent de fibroblastes, en particulier de fibroblastes humains.

12. Méthode selon l'une quelconque des revendications 10 et 11, dans laquelle les iPSCs dans l'étape a) dérivent de cellules d'un patient ayant une maladie neuromusculaire ou un trouble musculaire.

13. Méthode selon la revendication 12, dans laquelle la maladie neuromusculaire est une dystrophie musculaire choisie dans le groupe constitué par la dystrophie musculaire de Duchenne (DMD), la dystrophie myotonique (MD), la dystrophie facio-scapulo-humérale (FSHD) et la dystrophie musculaire scapulo-humérale de type 2A (LGMD2A).

14. Méthode selon l'une quelconque des revendications 1 à 13, dans laquelle le milieu de culture dans les étapes a) à e) est un milieu sans sérum.

15. Méthode selon l'une quelconque des revendications 1 à 14, dans laquelle les cellules sont cultivées dans les étapes a) à e) sur une surface revêtue de laminine, de collagène de type IV et de protéoglycanes.
